# EUROPEAN PATENT SPECIFICATION

(11) **EP 0 684 018 B1**
(45) Date of publication and mention of the grant of the patent: **17.10.2001**
(21) Application number: 94500090.9
(22) Date of filing: 23.05.1994
(51) Int. Cl.: A61B 17/60, A61B 17/58

(54) **A system for setting cervical vertebrae from behind**
System zur Halswirbelsetzung von der Rückseite
Système pour mettre en place les vertèbres cervicales par l'arrière

(43) Date of publication of application: 29.11.1995
(73) Proprietor: Barbera Alacreu, José Vicente, 46010 Valencia (ES)
(72) Inventor: Barbera Alacreu, José Vicente, 46010 Valencia (ES)
(74) Representative: Koepe, Gerd L., Dipl.-Chem.

(56) References cited:
- EP-A- 0 167 345
- DE-A- 1 958 429
- FR-A- 522 040
- FR-A- 2 697 428
- US-A- 3 858 578

## Description

The present invention relates to a system for setting cervical vertebrae from behind providing, in addition to the function for which it has been conceived, a number of advantages that shall be explained hereinafter and others that are inherent in its layout and construction.

Several systems designed specifically to set the cervical column from behind are currently known. The said conventional systems, being a part of the prior art, could be divided into five types listed below:
- Wires passing through holes made on the rear arch of each vertebra to be fixed that are then knotted to form a lacing that joins the vertebrae to one another. It is inconvenient in that the resulting set is not sufficiently solid, and if the wires are passed beneath the laminae manipulation can prove dangerous.
- Longitudinal bars joined to the vertebrae by means of wires passing beneath the rear arch laminae and then knotted around the bar. It is inconvenient in that the wires must pass beneath the laminae, and this is dangerous.
- Hooks catching hold of the vertebral laminae and then joined to each other by bars or screws. The Halifax system is inconvenient in that it is only useful to join two levels. The bar and hook system can only catch hold of the two end vertebrae and not the intermediate vertebrae. They cannot be used after laminectomy.
- Plates fixed to the vertebrae with screws that catch hold of the articular mass of each vertebra. It is inconvenient in that the place, being rigid, prefigures and conditions the place at which screws are inserted.
- The Magerl system combines the previous two systems and has a sublaminar hook and a plate joined to the hook that is screwed to one or two articular masses. It is inconvenient in that, being a rigid part, the position of the screws is prefigured and is further only useful to join two or three levels.

The Applicant has used his experience in the medical science subject hereof to design a new system for setting the cervical vertebrae from behind which conveniently overcomes the drawbacks that are usually found in the prior art methods succinctly described in the above section, now using hooks and plates and a number of jointed segments or cylinders crossed longitudinally and vertically to together establish a web housing a connecting wire that can be tightened and locked by a screw lying crosswise to the segment.

Hence, the invention concerns a system for setting cervical vertebrae from behind, characterized in comprising
(a) a group of distinct solid metal cylinders (1, 1a), said cylinders (1, 1a) having a stem of round cross-section and one of two possible heights, the top end of said cyclinders (1, 1a) being a hemisphere (2) having a diameter of 0.5 mm less than the diameter of the cross-section of said cylinders (1, 1a), whereas the lower end of said cylinders (1, 1a) is scooped to form a hemispherical cavity (3) whose internal diameter is 0.5 mm less than that of the cross-section of said cylinders (1, 1a) in order that the top protruding end (2) of one of said cylinders (1, 1a) may be precisely coupled to and turn within the lower scooped end (3) of another of said cylinders (1, 1a);
(b) said cylinders (1, 1a) having an axial circular bore (4) having a calibre of 2 mm, said axial circular bore (4) opening at the top and bottom ends of said cylinders (1, 1a) forming an internal duct, the top opening of said axial circular bore (4) being surrounded by four holes (5), said four holes (5) being arranged at the ends of a virtual cross, with connecting lines each being 4 mm long, and said top opening being at the intersection of said connecting lines; and
(c) the cylinder stem having at its longitudinal midpoint a threaded bore (6) with a calibre of 1.5 mm, said threaded bore (6) reaching the internal duct, said duct in the cylinders (1, 1a) allowing a number of said cylinders (1, 1a) to be strung by means of a braided wire (18), to form a structural assembly suited to the anatomic demands of the patient's vertebral morphology and size, the assembly being capable of being stiffened by tightening the said wire (18) using a wire tightening means, which couples the top and bottom ends of each of said cylinders (1, 1a);
(d) the final structure of the assembly being consolidated by setting each of said cylinders (1, 1a) one by one onto said wire (18), using small screws (7) inserted in the said threaded bore (6) provided in the stem of each of said cylinders (1, 1a), said screws (7) compressing the wire (18) in the internal duct, thereby preventing said cylinders (1, 1a) from slipping.

In a preferred system of the invention, one of said cylinders (1) having the larger height is 8 mm high, measured from the top end of said hemisphere (2) to the perimeter of said hemispheral cavity (3), and has a diameter of 5 mm, whereas another of said cylinders (1a) having the lower height is 4 mm high and has a diameter of 5 mm.

It is also preferred in accordance with the invention that one of said cylinders (1) having the larger height is a cylinder selected from
(a) one of said cylinders (1);
(b) one of said cylinders (1), additionally comprising a downward hook (10) projecting from the upper half of a side wall of the cylinder body at an upwardly open 45 ° angle to the longitudinal axis of the cylinder (1) and bending down until its final portion is parallel to the longitudinal axis of said cylinder (1);
(c) one of said cylinders (1), additionally comprising an upward hook (11) projecting from the lower half of a side wall of the cylinder body at a downwardly open 45° angle to the longitudinal axis of the cylinder (1) and
   bending up until its final portion is parallel to the longitudinal axis of said cylinder (1); and
(d) one of said cylinders (1), additionally comprising an articular screw plate (12) of trapeziodal shape projecting from a side wall of the cylinder body at the major axis plane of the cylinder (1) with a length of 15 mm and having the same heigth as the cylinder stem on the side adjacent to the cylinder (1) and a heigth of 8 mm on the side remote from the cylinder (1).

In an advantageous manner, the cylinder (1) having a downward hook (10) comprises the hook (10) as a 5 mm wide strip, and said threaded bore (6) for inserting said screws (7) is located on the side of said cylinder (1) diametrically opposite to the side from which the hook (10) projects.

In another advantageous embodiment, the cylinder (1) having an upward hook (11) comprises the hook (11) as a 5 mm wide strip, and said threaded bore (6) for inserting said screws (7) is located on the side of said cylinder (1) diametrically opposite to the side from which the hook (11) projects.

It is also an advantageous embodiment of the system that the cylinder (1) having an articular screw plate (12) has said plate (12) pierced by a hole (13) having a diameter of 3 mm, said hole (13) being located at a distance of 2 mm from the longer end of said plate (13) remote from the cylinder (1), and said cylinder (1) comprises an additional threaded bore (6) for inserting said screws (7), the two threaded bores (6) being located at right angles to said plate (12).

According to the invention, there is preferred a system further comprising
- a closing cylinder (15) having a height equal to said lower of said heights and comprising
   - a stem of round cross-section, and the top end of said closing cylinder (15) being a hemisphere (2) having a diameter of 0.5 mm less than the diameter of the cross-section of said closing cylinder (15);
   - an axial circular bore (4) having a calibre of 2 mm, said axial circular bore (4) opening at the top and bottom ends of said closing cylinder (15) forming an internal duct, the top opening of said axial circular bore (4) being surrounded by four holes (5), said four holes (5) being arranged at the ends of a virtual cross, with connecting lines each being 4 mm long, and said top opening being at the intersection of said connecting lines;
   - a flat lower end (16) for closing the system;
   - a dihedral notch (38) along the length of its stem, said notch (18a) leading towards the central axis; and
   - said axial circular bore (4) opens at the top end of said closing cylinder (15) and becomes said notch (38) in said stem of the closing cylinder (15).

In a furthermore preferred system,
(a) said braided wire (18) is a braided metal wire having a length of 40 cm and a thickness of 1.5 mm in order to pass freely through the axial circular bore (4) of said cylinders (1, 1a);
(b) said braided wire (18) is fixed at a first end to a circular plate (9) having a diameter of 4 mm and shaped as a spherical shell, the surface of which being adjusted to the top surface of the cylinders (1, 1a);
(c) said circular plate (9) comprises four spikes (19) each projecting, by a length of 1 mm, from the ends of two perpendicular diameters of said plate (9) so as to be housed in said four holes (5) surrounding said axial circular bore (4) at the top ends of said cylinders (1, 1a); and
(d) said braided wire (18), at its second end ends in a point (20) allowing said braided wire (18) to be easily threaded through said axial circular bore (4) of the cylinders (1, 1a).

One embodiment of the system of the invention is that the wire tightening means comprises a wrench having
(a) a first part (21) being a metal cylinder having a length of 20 cm and a diameter of 1 cm, a first end of said first part (21) having two curved sheets (22) being sized so as to be able to firmly hold between them the closing cylinder (15); the first part having a bore (23) having a calibre of 2 mm being provided on the surface of said first part (21) at the midpoint between said two curved sheets (22), said bore (23) extending along the stem of said first part (21) to surface again (25) 2 cm from the second end of said first part (21); a cylindrical scoop at the surface of second end of said first part (21), said scoop extending along the central axis down to the first end; and the circular perimeter of the surface at the second end being a cog-wheel (28); and
(b) the wrench having a second part (27) conformed as a hexagonal prism, having a stem (26) which can be housed in said cylinder scoop of the first part.

Even more preferred is a wrench wherein said second part (27), which is conformed as a hexagonal prism has a major diameter of 6 mm and a length of 3 cm, a 2 mm bore (29) appearing at a distance of 5 mm from its first end and extending upwards inside said prism to surface again on the same face, 1 cm towards the second end.

In an advantageous manner, the cylindrical stem (26), projecting from the first end of said prism (27) and fitting in the scoop provided therefor in the first, cylindrical part, crosses the full length of the first part, leaving through the first end of said first part (26a) and has a small boss that prevents vertical movement, thereby permanently fixing both first and second parts, and the second end of the prismatic part being a cog-wheel arranged to oppose the cogs on the first part.

It is also particularly preferred that said system additionally comprises a T-shaped torque wrench (30) having a hexagonal pitch (31) adapted to the prism (27) on the second part and allowing the same to turn about the first part clockwise, the return motion being prevented by the cog-wheels (28) on both abutting parts.

The system of the invention described above entails a number of advantages:
- Possibility of being used to join all the required levels;
- because it is jointed, neither the point of insertion of the screws nor the position of the hooks is predetermined, eliminating risks and expediting the placement and adaptation of the system to the morphology of the column;
- having screws and hooks, it can also be used after laminectomy.

The system of the present invention affords the foregoing advantages, in addition to others that may be easily be inferred from the embodiment described in detail hereinafter to expedite the understanding of the characteristics set out before, contemporaneously revealing a number of details. For this purpose, this specification has attached a number of drawings showing, for illustrative and non-limitative purposes, a practical embodiment of its object. In the drawings,
- Figures 1 and 2 show a side elevation view of two segments of cylinders being large and small, respectively;
- Figures 3, 4 and 5 are cross-sections of the said segments, viewed along A-A', B-B' and C-C';
- Figures 6, 7 and 8 show side elevation views of a type of cylinder having a downward hook;
- Figures 9, 10 and 11 show side elevation views of another type of cylinder having an upward hook;
- Figures 12, 13 and 14 show side elevation views of a third type of cylinder having a screw plate;
- Figure 15 illustrates a side elevation view of a closing cylinder;
- Figures 16 and 17 are cross-sections of the cylinder of the previous figure, seen along D-D' and E-E';
- Figure 18 shows the articular screw;

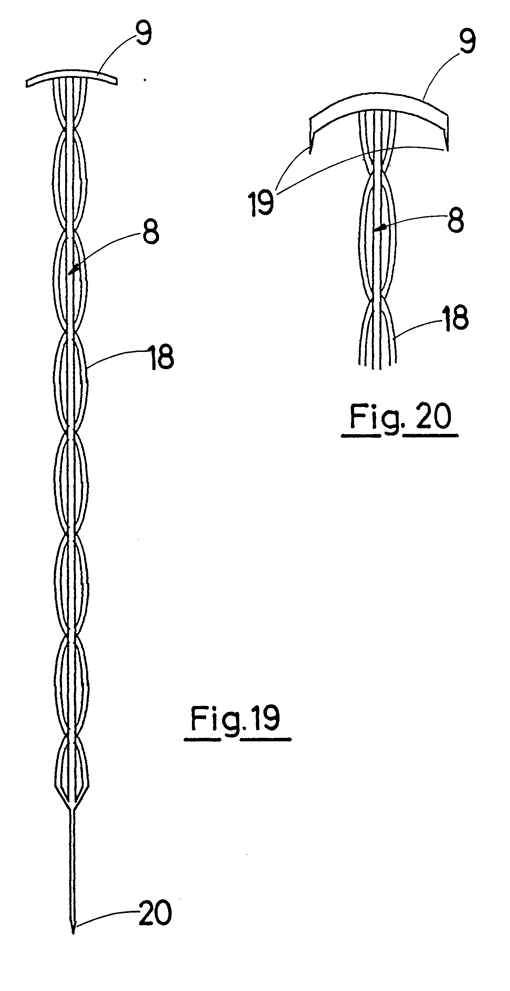

- Figures 19 and 20 are the internal wire and a close view of the wire plate;
- Figures 21, 22 and 23 show the wire tightening wrench, seen separately as a front elevation of the top and lower part, a side elevation thereof and a plan view of the top part;

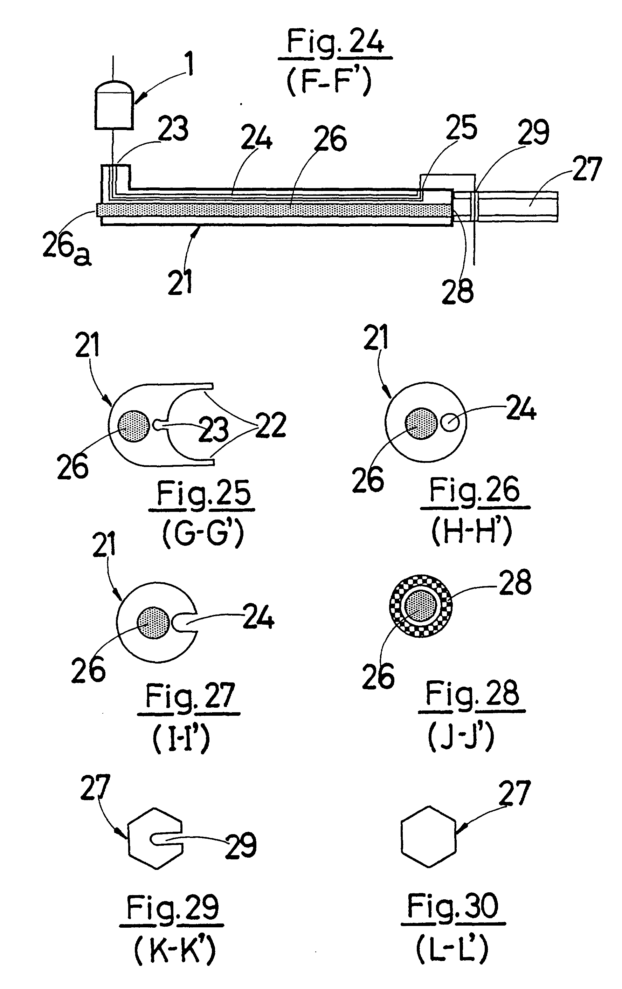

- Figure 24 is a longitudinal section of the lower part of the wire tightening wrench, seen along F-F';
- Figures 25, 26, 27, 28, 29 and 30 are the various sections of the lower part of the wire tightening wrench, seen along G-G', H-H', I-I', J-J', K-K' and L-L'; and

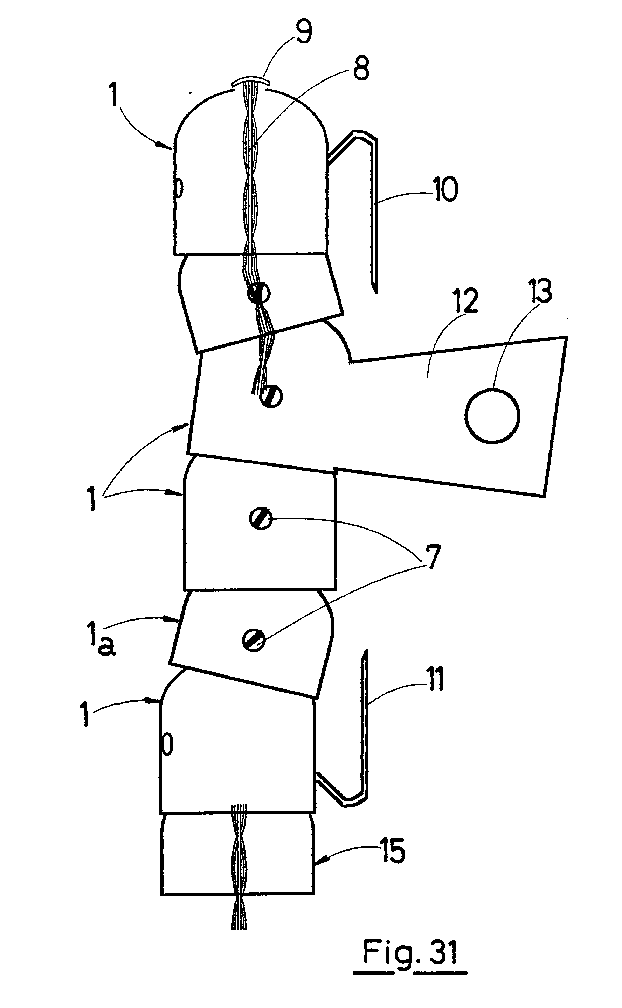

- Figure 31 shows an embodiment of the assembled, stiffened and coupled system with the incurvations imposed by the setting elements.

As shown in the drawings, the system for setting cervical vertebrae subject hereof, and in accordance with an embodiment thereof, comprises several distinct elements:

The first group of elements comprises different parts, that we shall call segments or cylinders.

Each of the segments is a solid metal cylinder 1 whose two ends are different.

The top end is a hemisphere 2 having a diameter that is 0.5 millimetres shorter than the diameter of the cross-section of the cylinder.

The lower end is scooped and forms a likewise hemispherical cavity 3 having an internal diameter that is 0.5 millimeters shorter than the diameter of the cylinder.

The reason for having this shape and these dimensions at the ends is that the top protruding end of a cylinder can be precisely coupled to and turn within the lower scooped end of another cylinder arranged above the same.

The whole cylinder is longitudinally crossed vertically by a hollow circular bore 4 having a 2 mm calibre, that opens at the top and bottom ends. This bore we shall refer to as cylinder web.

At the top end, surrounding the top opening of the circular bore, there are four small holes 5 arranged at the ends of a cross whose lines are each 4 millimetres long. The holes are for the wire 8 plate 9 to be fitted.

Halfway up the stem of the cylinder there is a threaded bore 6 with a 1.5 millimetre calibre, reaching the circular bore 4 or cylinder web. This bore shall house a small screw 7 whose blunt point shall, when fully screwed in, reach up to the opposite wall of the circular vertical bore of the cylinder. This is the screw that locks the wire 8.

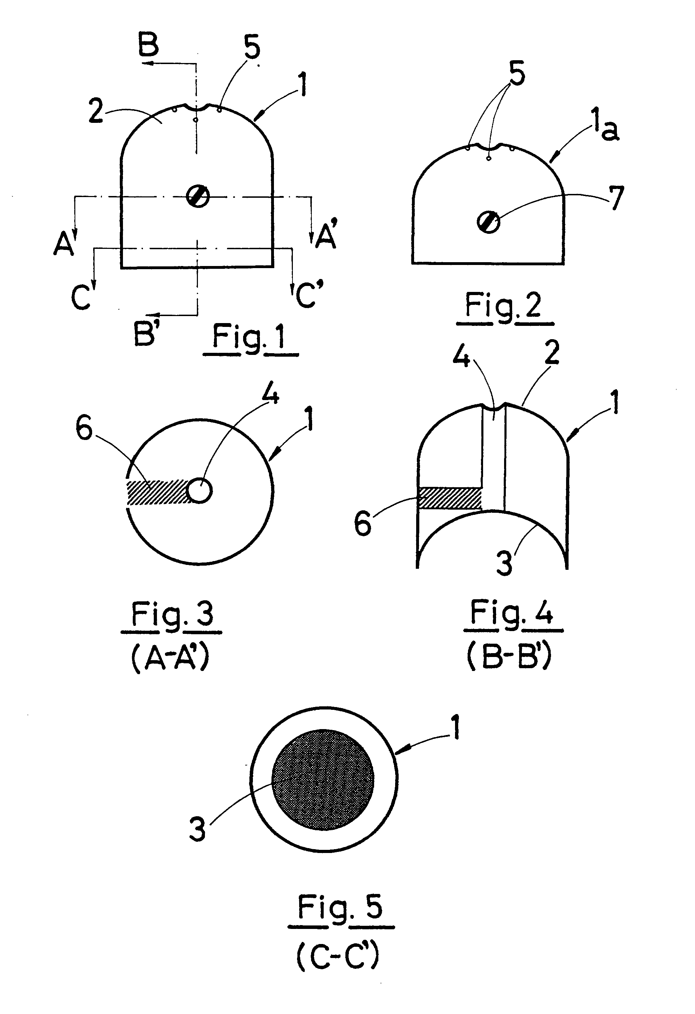

The cylinders are two in size:
- Large, as in Fig. 1, being e.g. 8 mm high, measured from the pole of the top hemisphere down to the perimeter of the hollow lower base, and being e.g. 5 mm in diameter.
- Small 1a, as in Fig. 2, being e.g. 4 mm high and 5 mm in diameter.

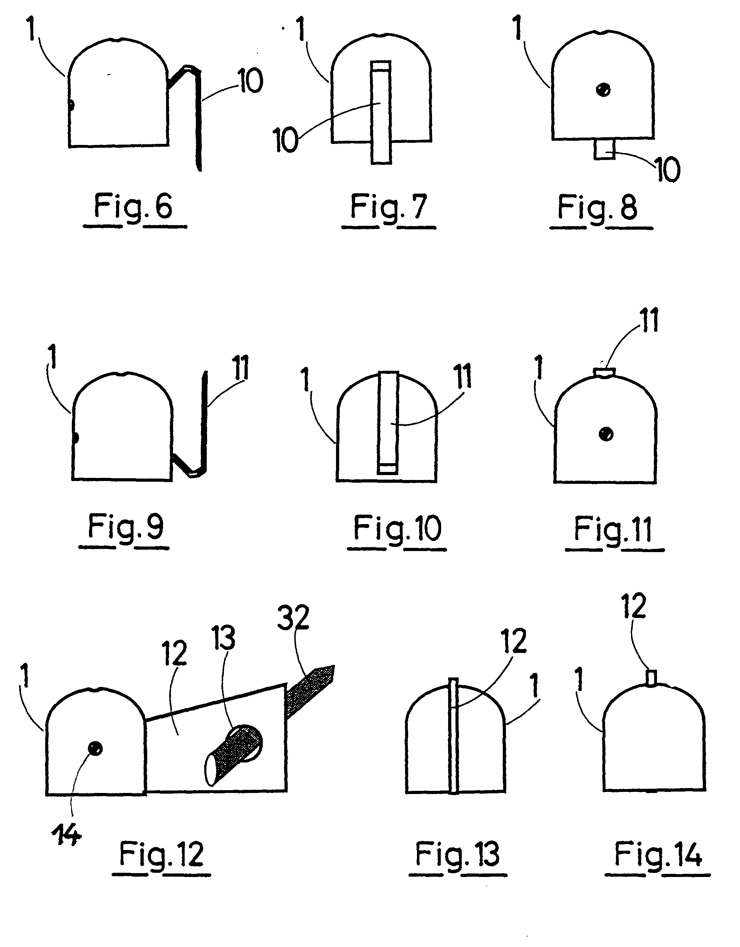

The large cylinders are divided into four types:
- SIMPLE: Comprising a simple cylinder as described in Figures 1 to 5.
- WITH DOWNWARD HOOK: This is a "simple" cylinder with a hook 10 projecting from one of its sides. This hook shall comprise a 5 mm wide sheet projecting from the top half of the cylinder body, at an upwardly open 45° angle to the longitudinal axis of the cylinder. The sheet then bends down until its final portion is parallel to the longitudinal axis of the cylinder. In this type, the wire locking screw bore lies at the side of the cylinder being diametrically opposite the projection of the hook, as shown in Figures 6 to 8.
- WITH UPWARD HOOK: This is similar to the above, though in this case the hook 11 projects from the lower half of the cylinder at a downwardly open 45° angle and the sheet then bends up. In this type, the wire locking screw bore lies at the side of the cylinder being diametrically opposite the projection of the hook, as shown in Figures 9 to 11.
- WITH ARTICULAR SCREW PLATE 32: A trapezoidal vertical plate 12 projects from the cylinder body at the major axis plane of the cylinder, extending sideways for e.g. 15 mm. The shorter side of the plate has the same length as the cylinder stem. The longer side of the plate measures e.g. 8 mm. The plate is crossed at 2 mm from its free end, its longer end, by a hole 13 having a diameter of e.g. 3 mm. In this type, two bores 14 are provided for the wire locking screw, lying on both the front and the rear faces of the cylinder, relative to the plate, as shown in Figures 12 to 14.

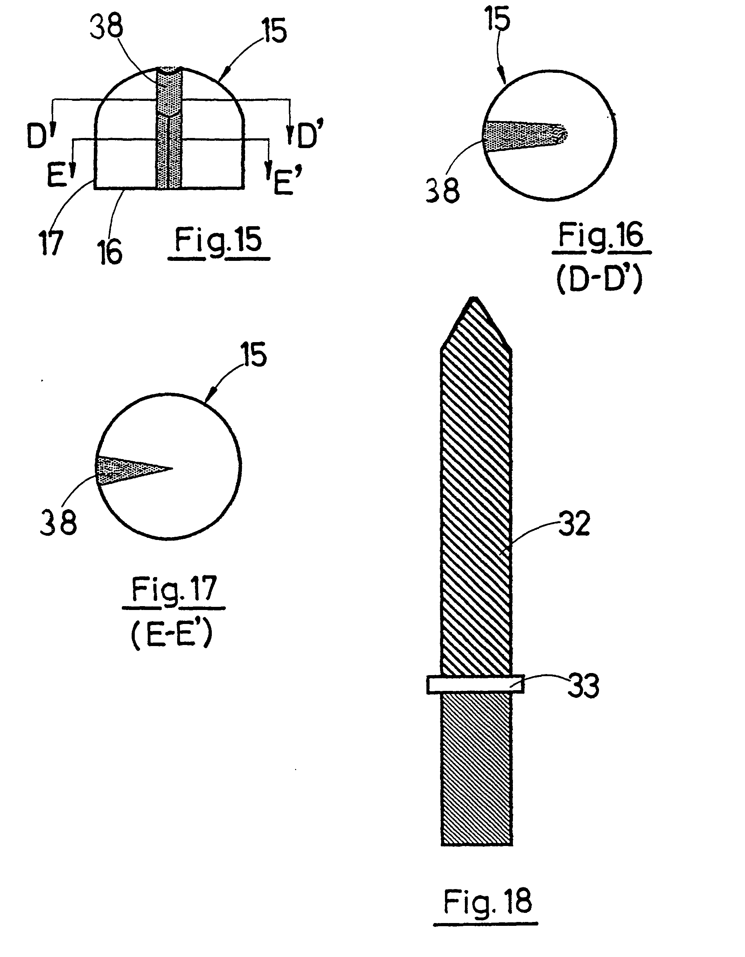

There are two types of small cylinders:
- SIMPLE: Carrying no hooks or plate, as in Fig. 2.
- CLOSING CYLINDER: This cylinder is numbered 15 and has a particular morphology. Its lower end is not scooped, and is flat instead as at 16. The stem 17 of the cylinder has a dihedral notch 38 along its length, leading towards the central axis. It carries no locking screw. The web, which opens at the top end, continues at the height of the cylindrical stem with the inner angle of the notch as in Figures 15 to 17.

The second group of elements comprises the internal wire 8 and its components:

The components are:
- A braided metal wire 18 being e.g. 40 cm long and e.g. 1.5 mm thick, being therefore able to pass freely through the circular bore 4 or hollow web of the segments.
- At one end the wire is fixed to a circular plate 9 having a diameter of e.g. 4 mm, and shaped as a spherical shell, the surface of which adjusts to the top end surface of the cylinder. This is the wire plate. Four spikes 19 being each e.g. 1 mm long, project from the ends of two perpendicular diameters of this plate, which shall be housed in the four bores 5 at the top end of the cylinder 1.
- The other end of the wire ends in a point 20 that allows the same to be easily threaded through the circular bore 4 or web of the segments. This is the wire point.

The third element is the wire tightening wrench.

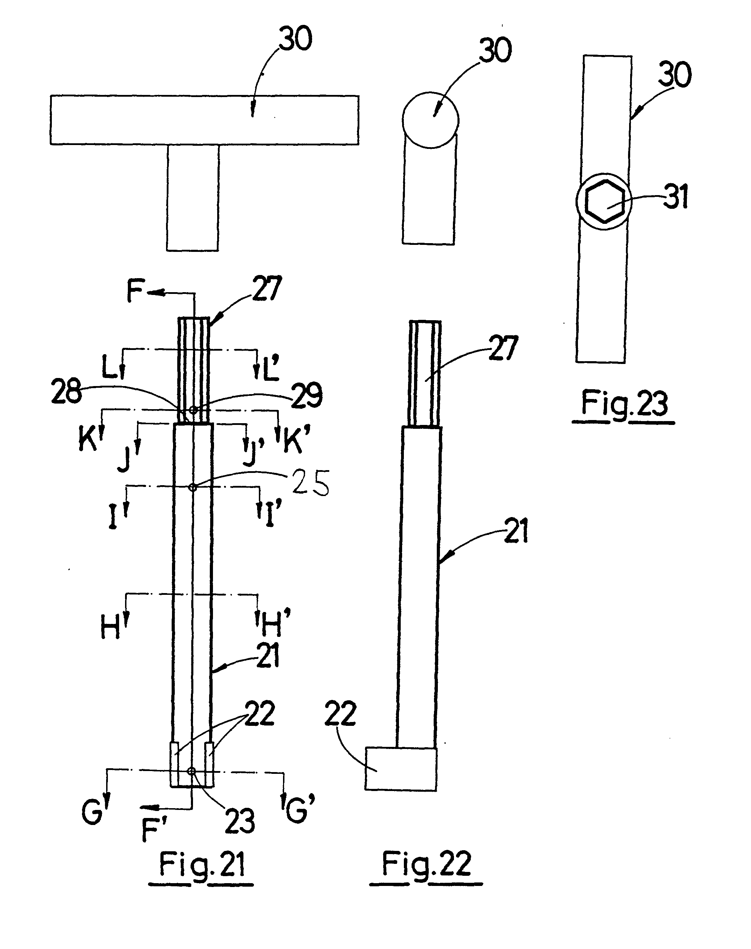

Such comprises three parts, as in Figures 21 to 23. The lower part is a metal cylinder being e.g. 20 cm long and e.g. 1 cm in diameter. Its lower end has two curved sheets 22 being sized so as to be able to firmly couple between the same the closing cylinder.

A 2 mm bore 23 is provided on the surface of the cylinder at the midpoint between these two sheets which extends upwards as at 24 along the stem of the cylinder to again surface at 25, 2 cm from the top end.

The surface of the top end has a cylindrical scoop that extends along the central axis down to the lower end. This cavity houses the stem 26 of the top part 27.

The circular perimeter of the top surface is a cog-wheel 28 with triangular rectangular cogs.

The top part 27 is a hexagonal prism 27 with a major diameter of e.g. 6 mm and a length of e.g. 3 cm A 2 mm bore 29 appears at a distance of e.g. 5 mm from its lower end, extending upwards inside the prism 27 to surface again on the same face, e.g. 1 cm higher. A cylindrical stem, 26 projects from the lower end of the prism 27, which fits in the cavity provided therefor in the lower part, crossing its full length, leaving through the lower end of such part, as at 26a. At its outlet from the lower end of the lower part, the stem has a small boss that prevents vertical movement, thereby permanently fixing both parts.

The lower end of the top part or prism 27 is a cog-wheel, with triangular rectangular cogs arranged inversely to the cogs on the lower part.

A T shaped torque wrench 30 with a hexagonal pitch 31 adapting to the prism 27 on the top part and allowing to same to turn about the lower part clockwise. The return motion is prevented by the cog-wheels on both abutting parts.

After exposing the rear arches of the vertebral segments that are to be merged, a decision is made as to the type of setting elements that are to be used for each level, hooks or screws, and the respective distances at which these elements shall have to be positioned are calculated.

At the levels where screws 32 are to be used, these are placed directly on the articular masses, introducing the same to the limit and without any limitations as to their position.

The various cylinders 1, 12, 15 having the required setting elements (upward facing hook 11, downward facing hook 10, screw plate 12) and the simple cylinders of different sizes are strung in the braided wire 8, 18. With the various sizes of simple cylinders 1, 1a the assembly shall be adjusted to the anatomic demands required by the vertebral morphology and size.

The hooks are placed on the laminae and for the levels in which a screw 32 has been inserted, a cylinder and a plate 12 are placed. The projecting stem of the screw 32 is inserted through the bore in the plate 12 and the nut attaching the plate to the screw is then placed. As aforesaid, the distance between setting elements is covered with like elements.

The last element strung in the wire is a closing cylinder 15.

After the system is all assembled the central wire 8 is tightened using the appropriate wrench described in the apparatus section, and the system is thereby stiffened, adjusting to the incurvations imposed by the setting elements. Coupling is achieved due to the turning capacity the hemispherical arrangement at the top and lower ends of each cylinder provides.

When the central wire is tightened to the limit, before removing the tightening wrench, the final position is consolidated by screwing the small screws 7 with which each cylinder is provided, compressing the wire in the central duct, preventing it from sliding.

The tightening wrench is removed and forceps are used to tighten the closing cylinder 15 to the wire, solidly fixing the whole system.

The closing cylinder 15 is coupled between the sheets 22 of the bottom end of the wrench 21.

The tightening wire is placed in the dihedral notch 38 of the closing cylinder and is then inserted through the lower bore of the lower part until it leaves through the top bore.

The wire is then inserted through the lower bore of the top part until it leaves through the top bore.

At this point, the torque wrench is used to turn the top part about the lower part, in such a way that the free end of the wire is wound around the prism and is tightened until the torque wrench stops turning.

The tension applied to the wire is preserved because the two cog-wheels at the top and bottom ends of the two parts prevent a turn in the direction opposite the direction in which force is applied.

With the wrench in position, the locking screws in each cylinder are gradually tightened, starting with the top cylinder and moving down towards the bottom cylinder.

After tightening each locking screw it is found that the torque wrench cannot turn, viz. it is found that the wire has lost no tightness.

Once all the locking screws are tightened, the tightening wrench is removed and suitable forceps are used to tighten the closing cylinder 15 in order for the dihedral notch 38 to close upon the wire 8,18 preventing it from slipping or becoming loose.

## Claims

1. A system for setting cervical vertebrae from behind, **characterized in** comprising
(a) a group of distinct solid metal cylinders (1, 1a), said cylinders (1, 1a) having a stem of round cross-section and one of two possible heights, the top end of said cyclinders (1, 1a) being a hemisphere (2) having a diameter of 0.5 mm less than the diameter of the cross-section of said cylinders (1, 1a), whereas the lower end of said cylinders (1, 1a) is scooped to form a hemispherical cavity (3) whose internal diameter is 0.5 mm less than that of the cross-section of said cylinders (1, 1a) in order that the top protruding end (2) of one of said cylinders (1, 1a) may be precisely coupled to and turn within the lower scooped end (3) of another of said cylinders (1, 1a);
(b) said cylinders (1, 1a) having an axial circular bore (4) having a calibre of 2 mm, said axial circular bore (4) opening at the top and bottom ends of said cylinders (1, 1a) forming an internal duct, the top opening of said axial circular bore (4) being surrounded by four holes (5), said four holes (5) being arranged at the ends of a virtual cross, with connecting lines each being 4 mm long, and said top opening being at the intersection of said connecting lines; and
(c) the cylinder stem having at its longitudinal midpoint a threaded bore (6) with a calibre of 1.5 mm, said threaded bore (6) reaching the internal duct, said duct in the cylinders (1, 1a) allowing a number of said cylinders (1, 1a) to be strung by means of a braided wire (18), to form a structural assembly suited to the anatomic demands of the patient's vertebral morphology and size, the assembly being capable of being stiffened by tightening the said wire (18) using a wire tightening means, which couples the top and bottom ends of each of said cylinders (1, 1a);
(d) the final structure of the assembly being consolidated by setting each of said cylinders (1, 1a) one by one onto said wire (18), using small screws (7) inserted in the said threaded bore (6) provided in the stem of each of said cylinders (1, 1a), said screws (7) compressing the wire (18) in the internal duct, thereby preventing said cylinders (1, 1a) from slipping.

2. The system as in claim 1, wherein one of said cylinders (1) having the larger height is 8 mm high, measured from the top end of said hemisphere (2) to the perimeter of said hemispheral cavity (3), and has a diameter of 5 mm, whereas another of said cylinders (1a) having the lower height is 4 mm high and has a diameter of 5 mm.

3. The system as in claim 1 or claim 2, wherein one of said cylinders (1) having the larger height is a cylinder selected from
(a) one of said cylinders (1);
(b) one of said cylinders (1), additionally comprising a downward hook (10) projecting from the upper half of a side wall of the cylinder body at an upwardly open 45 ° angle to the longitudinal axis of the cylinder (1) and bending down until its final portion is parallel to the longitudinal axis of said cylinder (1);
(c) one of said cylinders (1), additionally comprising an upward hook (11) projecting from the lower half of a side wall of the cylinder body at a downwardly open 45 ° angle to the longitudinal axis of the cylinder (1) and bending up until its final portion is parallel to the longitudinal axis of said cylinder (1); and
(d) one of said cylinders (1), additionally comprising an articular screw plate (12) of trapeziodal shape projecting from a side wall of the cylinder body at the major axis plane of the cylinder (1) with a length of 15 mm and having the same heigth as the cylinder stem on the side adjacent to the cylinder (1) and a heigth of 8 mm on the side remote from the cylinder (1).

4. The system as in claim 3, wherein the cylinder (1) having a downward hook (10) comprises the hook (10) as a 5 mm wide strip, and said threaded bore (6) for inserting said screws (7) is located on the side of said cylinder (1) diametrically opposite to the side from which the hook (10) projects.

5. The system as in claim 3, wherein the cylinder (1) having an upward hook (11) comprises the hook (11) as a 5 mm wide strip, and said threaded bore (6) for inserting said screws (7) is located on the side of said cylinder (1) diametrically opposite to the side from which the hook (11) projects.

6. The system as in claim 3, wherein the cylinder (1) having an articular screw plate (12) has said plate (12) pierced by a hole (13) having a diameter of 3 mm, said hole (13) being located at a distance of 2 mm from the longer end of said plate (13) remote from the cylinder (1), and said cylinder (1) comprises an additional threaded bore (6) for inserting said screws (7), the two threaded bores (6) being located at right angles to said plate (12).

7. The system as in claims 1 to 6, further comprising
- a closing cylinder (15) having a height equal to said lower of said heights and comprising
- a stem of round cross-section, and the top end of said closing cylinder (15) being a hemisphere (2) having a diameter of 0.5 mm less than the diameter of the cross-section of said closing cylinder (15);
- an axial circular bore (4) having a calibre of 2 mm, said axial circular bore (4) opening at the top and bottom ends of said closing cylinder (15) forming an internal duct, the top opening of said axial circular bore (4) being surrounded by four holes (5), said four holes (5) being arranged at the ends of a virtual cross, with connecting lines each being 4 mm long, and said top opening being at the intersection of said connecting lines;
- a flat lower end (16) for closing the system;
- a dihedral notch (38) along the length of its stem, said notch (18a) leading towards the central axis; and
- said axial circular bore (4) opens at the top end of said closing cylinder (15) and becomes said notch (38) in said stem of the closing cylinder (15).

8. The system as in claims 1 to 7, wherein
(a) said braided wire (18) is a braided metal wire having a length of 40 cm and a thickness of 1.5 mm in order to pass freely through the axial circular bore (4) of said cylinders (1, 1a);
(b) said braided wire (18) is fixed at a first end to a circular plate (9) having a diameter of 4 mm and shaped as a spherical shell, the surface of which being adjusted to the top surface of the cylinders (1, 1a);
(c) said circular plate (9) comprises four spikes (19) each projecting, by a length of 1 mm, from the ends of two perpendicular diameters of said plate (9) so as to be housed in said four holes (5) surrounding said axial circular bore (4) at the top ends of said cylinders (1, 1a); and
(d) said braided wire (18), at its second end ends in a point (20) allowing said braided wire (18) to be easily threaded through said axial circular bore (4) of the cylinders (1, 1a).

9. The system as in claim 7, wherein the wire tightening means comprises a wrench having
(a) a first part (21) being a metal cylinder having a length of 20 cm and a diameter of 1 cm, a first end of said first part (21) having two curved sheets (22) being sized so as to be able to firmly hold between them the closing cylinder (15); the first part having a bore (23) having a calibre of 2 mm being provided on the surface of said first part (21) at the midpoint between said two curved sheets (22), said bore (23) extending along the stem of said first part (21) to surface again (25) 2 cm from the second end of said first part (21); a cylindrical scoop at the surface of second end of said first part (21), said scoop extending along the central axis down to the first end; and the circular perimeter of the surface at the second end being a cog-wheel (28); and
(b) the wrench having a second part (27) conformed as a hexagonal prism, having a stem (26) which can be housed in said cylinder scoop of the first part.

10. The system as in claim 9, wherein said second part (27), which is conformed as a hexagonal prism has a major diameter of 6 mm and a length of 3 cm, a 2 mm bore (29) appearing at a distance of 5 mm from its first end and extending upwards inside said prism to surface again on the same face, 1 cm towards the second end.

11. The system as in claim 9 or claim 10, wherein the cylindrical stem (26), projecting from the first end of said prism (27) and fitting in the scoop provided therefor in the first, cylindrical part, crosses the full length of the first part, leaving through the first end of said first part (26a) and has a small boss that prevents vertical movement, thereby permanently fixing both first and second parts, and the second end of the prismatic part being a cog-wheel arranged to oppose the cogs on the first part.

12. The system as in claims 9 or 10, said system additionally comprising a T-shaped torque wrench (30) having a hexagonal pitch (31) adapted to the prism (27) on the second part and allowing the same to turn about the first part clockwise, the return motion being prevented by the cog-wheels (28) on both abutting parts.

## Patentansprüche

1. System zum Setzen zervikaler Wirbel von hinten, **dadurch gekennzeichnet, daß** es umfaßt:
(a) eine Gruppe von verschiedenen festen Metall-Zylindern (1, 1a), wobei die Zylinder (1, 1a) einen Stiel mit rundem Querschnitt und eine von zwei möglichen Höhen haben, das obere Ende der Zylinder (1, 1a) eine Halbkugel (2) mit einem Durchmesser ist, der um 0,5 mm geringer als der Durchmesser des Querschnitts der Zylinder (1, 1a) ist, während das untere Ende der Zylinder (1, 1a) ausgehöhlt ist und einen halbkugelförmigen Hohlraum (3) bildet, dessen Innendurchmesser um 0,5 mm geringer ist als derjenige des Querschnitts der Zylinder (1, 1a), damit das obere vorstehende Ende (2) eines der Zylinder (1, 1a) präzise in das untere ausgehöhlte Ende (3) eines anderen der Zylinder (1, 1a) eingekoppelt werden kann und sich in diesem drehen kann;
(b) wobei die Zylinder (1, 1a) eine axiale kreisförmige Bohrung (4) mit einer Weite von 2 mm aufweisen, wobei die axiale kreisförmige Bohrung (4) am oberen und unteren Ende der Zylinder (1, 1a) offen ist und einen Innengang bildet, wobei die obere Öffnung der axialen kreisförmigen Bohrung (4) von vier Löchern (5) umgeben ist und diese vier Löcher (5) an den Enden eines virtuellen Kreuzes angeordnet sind und die Verbindungslinien jeweils 4 mm lang sind, und wobei die obere Öffnung am Schnittpunkt der Verbindungslinien angeordnet ist; und
(c) wobei der Stiel der Zylinder an seinem Längs-Mittelpunkt eine mit einem Gewinde versehene Bohrung (6) mit einer Weite von 1,5 mm aufweist, wobei die mit einem Gewinde versehene Bohrung (6) den Innengang erreicht, wobei der Gang in den Zylindern (1, 1a) ermöglicht, daß eine Anzahl der Zylinder (1, 1a) mittels eines geflochtenen Drahts (18) unter Bildung einer Strukturanordnung aufgefädelt werden kann, die den anatomischen Erfordernissen der Morphologie und Größe der Wirbelsäule eines Patienten angepaßt ist, wobei die Anordnung dadurch versteift werden kann, daß man den Draht (18) unter Verwendung einer Einrichtung zum-Verspannen verspannt, was das obere und das untere Ende jedes der Zylinder (1, 1a) verbindet;
(d) wobei die End-Struktur der Anordnung dadurch gestärkt wird, daß man jeden der Zylinder (1, 1a) einen nach dem anderen auf den Draht (18) setzt, daß man kleine Schrauben (7) verwendet, die in die mit einem Gewinde versehene Bohrung (6), die in dem Stiel jedes der Zylinder (1, 1a) vorgesehen sind, eingesetzt werden, wobei die Schrauben (7) den Draht (18) in dem Innengang zusammendrücken und dadurch verhindern, daß die Zylinder (1, 1a) rutschen.

2. System nach Anspruch 1, worin einer der Zylinder (1), der die größere Höhe hat, 8 mm hoch ist, gemessen vom oberen Ende der Halbkugel (2) zum Umfang des halbkugelförmigen Hohlraums (3), und einen Durchmesser von 5 mm hat, während ein anderer der Zylinder (1a), der die geringere Höhe hat, 4 mm hoch ist und eine Durchmasser von 5 mm hat.

3. System nach Anspruch 1 oder Anspruch 2, worin einer der Zylinder (1), der die größere Höhe hat, ein Zylinder ist, der gewählt ist aus
(a) einem der Zylinder (1);
(b) einem der Zylinder (1), der zusätzlich einen nach unten gerichteten Haken (10) aufweist, der von der oberen Hälfte einer Seitenwand des Zylinderkörpers in einem nach oben offenen Winkel 45 °-Winkel zur Längsachse des Zylinders (1) vorsteht und nach unten gebogen ist, bis sein Endabschnitt parallel zur Längsachse des Zylinders (1) ist;
(c) einem der Zylinder (1), der zusätzlich einen nach oben gerichteten Haken (11) aufweist, der von der unteren Hälfte einer Seitenwand des Zylinderkörpers in einem nach unten offenen Winkel 45 °-Winkel zur Längsachse des Zylinders (1) vorsteht und nach oben gebogen ist, bis sein Endabschnitt parallel zur Längsachse des Zylinders (1) ist; und
(d) einem der Zylinder (1), der zusätzlich eine einen knickaufweisende Schraub-Platte (12) in Trapez-Form umfaßt, die von einer Seitenwand des Zylinderkörpers in der Hauptachsen-Ebene des Zylinders (1) mit einer Länge von 15 mm vorsteht und dieselbe Höhe wie der Stiel des Zylinders auf der dem Zylinder (1) benachbarten Seite und eine Höhe von 8 mm auf der von dem Zylinder (1) entfernten Seite aufweist.

4. System nach Anspruch 3, worin der Zylinder (1), der einen nach unten gerichteten Haken (10) aufweist, den Haken (10) in Form eines 5 mm breiten Streifens umfaßt und worin die mit einem Gewinde versehene Bohrung (6) zum Einsetzen der Schrauben (7) auf der Seite des Zylinders (1) angeordnet ist, die der Seite diametral gegenüber liegt, von der der Haken (10) vorsteht.

5. System nach Anspruch 3, worin der Zylinder (1), der einen nach oben gerichteten Haken (11) aufweist, den Haken (11) in Form eines 5 mm breiten Streifens umfaßt und worin die mit einem Gewinde versehene Bohrung (6) zum Einsetzen der Schrauben (7) auf der Seite des Zylinders (1) angeordnet ist, die der Seite diametral gegenüber liegt, von der der Haken (11) vorsteht.

6. System nach Anspruch 3, worin bei dem Zylinder (1), der eine einen knickaufweisende Schraubplatte (12) aufweist, die Platte (12) von einem Loch (13) mit einem Durchmesser von 3 mm durchbohrt ist, wobei das Loch (13) in einem Abstand von 2 mm von dem längeren, von dem Zylinder (1) entfernt angeordneten Ende der Platte (12) angeordnet ist und der Zylinder (1) eine zusätzliche, mit einem Gewinde versehene Bohrung (6) zum Einsetzen der Schrauben (7) umfaßt, wobei die beiden mit einem Gewinde versehenen Bohrungen (6) in rechten Winkeln zu der Platte (12) angeordnet sind.

7. System nach den Ansprüchen 1 bis 6, umfassend weiter
- einen Schließzylinder (15), der eine Höhe aufweist, die gleich der geringeren der Höhen ist, und umfaßt
- einen Stiel mit rundem Querschnitt, wobei das obere Ende des Schließzylinders (15) eine Halbkugel (2) ist, die einen Durchmesser aufweist, der um 0,5 mm geringer ist als der Durchmesser des Querschnitts des Schließzylinders (15);
- eine axiale kreisförmige Bohrung (4) mit einer Weite von 2 mm, wobei sich die axiale kreisförmige Bohrung (4) am oberen und am unteren Ende des Schließzylinders (15) öffnet und einen Innengang bildet, wobei die obere Öffnung der axialen kreisförmigen Bohrung (4) von vier Löchern (5) umgeben ist und die vier Löcher (5) an den Enden eines virtuellen Kreuzes angeordnet sind und die Verbindungslinien jeweils 4 mm lang sind, und wobei die obere Öffnung am Schnittpunkt der Verbindungslinien angeordnet ist;
- ein flaches unteres Ende (16) zum Schließen des Systems;
- eine zweiflächige Kerbe (38) entlang der Länge des Stiels des Zylinders, wobei die Kerbe (38) in Richtung auf die zentrale Achse führt; und
- wobei sich die axiale kreisförmige Bohrung (4) am oberen Ende des Schließzylinders (15) öffnet und in dem Stiel des Schließzylinders (15) zu der Kerbe (38) wird.

8. System nach den Ansprüchen 1 bis 7, worin
(a) der geflochtene Draht (18) ein geflochtener Metalldraht mit einer Länge von 40 cm und einer Dicke von 1,5 mm ist, damit er frei durch die axiale kreisförmige Bohrung (4) der Zylinder (1, 1a) paßt;
(b) worin der geflochtene Draht (18) an einem ersten Ende an einer kreisförmigen Platte (9) befestigt ist, die einen Durchmesser von 4 mm hat und als Kugelschale geformt ist, deren Oberfläche an die obere Oberfläche der Zylinder (1, 1a) angepaßt ist;
(c) worin die kreisförmige Platte (9) vier Dorne (19) umfaßt, von denen jeder mit einer Länge von 1 mm von den Enden von zwei in rechtem Winkel angeordneten Durchmessern der Platte (9) vorsteht, so daß sie in den vier Löchern (5), die die axiale kreisförmige Bohrung (4) an den oberen Enden der Zylinder (1, 1a) umgeben, untergebracht werden; und
(d) worin der geflochtene Draht (18) an seinem zweiten Ende in einem Punkt (20) endet, der es ermöglicht, daß der geflochtene Draht (18) leicht durch die axiale kreisförmige Bohrung (4) der Zylinder (1, 1a) gefädelt werden kann.

9. System nach Anspruch 7, worin die Einrichtung zum Verspannen des Drahts einen Schraubenschlüssel umfaßt, der aufweist:
(a) einen ersten Teil (21), der ein Metallzylinder ist, der eine Länge von 20 cm und einen Durchmesser von 1 cm aufweist, wobei ein erstes Ende des ersten Teils (21) zwei gebogene Platten (22) aufweist, die ein solches Maß haben, daß sie zwischen sich den Schließzylinder (15) fest halten können, wobei der erste Teil (21) eine Bohrung (23) mit einer Weite von 2 mm aufweist, die auf der Oberfläche des ersten Teils (21) an dem Punkt mitten zwischen den beiden gebogenen Platten (22) vorgesehen ist, wobei sich die Bohrung (23) entlang des Stiels des ersten Teils (21) erstreckt und 2 cm entfernt von dem zweiten Ende des ersten Teils (21) wieder an die Oberfläche (25) kommt; eine zylindrische Höhlung an der Oberfläche des zweiten Endes des ersten Teils (21), wobei sich die Höhlung entlang der zentralen Achse hinab zu dem ersten Ende erstreckt; und wobei der Kreisumfang an dem zweiten Ende ein Zahnrad (28) ist; und
(b) wobei der Schraubenschlüssel einen zweiten Teil (27) aufweist, der an die Form eines hexagonales Prismas angepaßt ist und einen Stiel (26) aufweist, der in der zylindrischen Höhlung des ersten Teils untergebracht werden kann.

10. System nach Anspruch 9, worin der zweite Teil (27), der an die Form eines hexagonales Prisma angepaßt ist, einen Hauptdurchmesser von 6 mm und eine Länge von 3 cm hat, eine 2 mm starke Bohrung (29) aufweist, die in einem Abstand von 5 mm von seinem ersten Ende erscheint und sich innerhalb des Prismas nach oben erstreckt und auf derselben Fläche 1 cm von dem zweiten Ende entfernt wieder an die Oberfläche kommt.

11. System nach Anspruch 9 oder Anspruch 10, worin der zylindrische Stiel (26), der von dem ersten Ende des Prismas (27) vorsteht und in die Höhlung paßt, die dafür in dem ersten zylindrischen Teil vorgesehen ist, die volle Länge des ersten Teils kreuzt, durch das erste Ende des ersten Teils (26a) hindurch reicht und einen kleinen Wulst aufweist, der ein vertikales Bewegen verhindert und dadurch sowohl den ersten als auch den zweiten Teil permanent fixiert, und wobei das zweite Ende des wie ein Prisma geformten Teils ein Zahnrad ist, das so angeordnet ist, daß es den Zähnen an dem ersten Teil gegenüber liegt.

12. System nach Anspruch 9 oder 10, wobei das System zusätzlich einen T-förmigen Drehmoment-Schlüssel (30) umfaßt, der eine hexagonale Vertiefung (31) aufweist, die an das Prisma (27) an dem zweiten Teil angepaßt ist und es ermöglicht, daß sich dieses um den ersten Teil im Uhrzeigersinn dreht, wobei die Rückbewegung durch die Zahnräder (28) an beiden vorspringenden Teilen verhindert wird.

## Revendications

1. Système de positionnement des vertèbres cervicales par derrière, **caractérisé en ce qu'**il comprend :
(a) un groupe de cylindres métalliques solides distincts (1, 1a), lesdits cylindres (1, 1a) présentant une tige de section transversale arrondie et l'une de deux hauteurs possibles, l'extrémité supérieure desdits cylindres (1, 1a) étant une hémisphère (2) présentant un diamètre inférieur de 0,5 mm au diamètre de la section transversale desdits cylindres (1, 1a), tandis que l'extrémité inférieure desdits cylindres (1, 1a) a la forme d'une coupelle pour former une cavité hémisphérique (3) dont le diamètre interne est inférieur de 0,5 mm à celui de la section transversale desdits cylindres (1, 1a), afin que l'extrémité supérieure saillante (2) de l'un desdits cylindres (1, 1a) puisse être couplée avec précision sur, et tourner avec l'extrémité inférieure en forme de coupelle (3) d'un autre desdits cylindres (1, 1a) ;
(b) lesdits cylindres (1, 1a) présentant un alésage circulaire axial (4) présentant un calibre de 2 mm, ledit alésage circulaire axial (4) débouchant au niveau des extrémités inférieure et supérieure desdits cylindres (1, 1a) pour former un conduit intérieur, l'ouverture supérieure dudit alésage circulaire axial (4) étant entouré de quatre trous (5), lesdits quatre trous (5) étant agencés au niveau des extrémités d'une croix virtuelle, les lignes de raccordement ayant chacune une longueur de 4 mm, et ladite ouverture supérieure se trouvant au niveau de l'intersection desdites lignes de raccordement ; et
(c) la tige du cylindre présentant, en son point médian. dans le sens de la longueur, un alésage fileté (6) d'un calibre de 1,5 mm, ledit alésage fileté (6) atteignant le conduit intérieur, ledit conduit des cylindres (1, 1a) permettant à un certain nombre desdits cylindres (1, 1a) d'être attachés au moyen d'un fil tressé (18) pour former un ensemble structurel qui convient aux exigences anatomiques de la taille et de la morphologie vertébrale du patient, l'ensemble pouvant être renforcé en serrant ledit fil (18) et en utilisant un moyen de serrage du fil , qui couple les extrémités inférieure et supérieure de chacun desdits cylindres (1, 1a) ;
(d) la structure finale de l'ensemble étant consolidée en fixant chacun desdits cylindres (1, 1a) un par un sur ledit fil (18), en utilisant de petites vis (7) insérées dans ledit alésage fileté (6) et prévues dans la tige de chacun desdits cylindres (1, 1a), lesdites vis (7) comprimant le fil (18) dans le conduit intérieur, ce qui empêche lesdits cylindres (1, 1a) de glisser.

2. Système selon la revendication 1, dans lequel l'un desdits cylindres (1) présentant la plus grande hauteur est haut de 8 mm, mesurée entre l'extrémité supérieure de ladite hémisphère (2) et le périmètre de ladite cavité hémisphérique (3), et présente un diamètre de 5 mm, tandis qu'un autre desdits cylindres (1a) présentant la plus petite hauteur est haut de 4 mm et présente un diamètre de 5 mm.

3. Système selon la revendication 1 ou la revendication 2, dans lequel l'un desdits cylindres (1) présentant la plus grande hauteur est un cylindre choisi parmi :
(a) l'un desdits cylindres (1) ;
(b) l'un desdits cylindres (1), comprenant de plus un crochet dirigé vers le bas (10) qui fait saillie à partir de la moitié supérieure d'une paroi latérale du corps du cylindre suivant un angle de 45° ouvert vers le haut par rapport à l'axe longitudinal du cylindre (1) et étant recourbé vers le bas jusqu'à ce que sa partie finale soit parallèle à l'axe longitudinal dudit cylindre (1) ;
(c) l'un desdits cylindres (1), comprenant en outre un crochet dirigé vers le haut (11) qui fait saillie à partir de la moitié inférieure d'une paroi latérale du corps du cylindre suivant un angle de 45° ouvert vers le bas par rapport à l'axe longitudinal du cylindre (1) et étant recourbé vers le haut jusqu'a ce que sa partie finale soit parallèle à l'axe longitudinal dudit cylindre (1) ;
(d) l'un desdits cylindres (1), comprenant en outre une plaque à vis articulaire (12) de forme trapézoïdale qui fait saillie à partir d'une paroi latérale du corps du cylindre au niveau du plan principal de l'axe du cylindre (1), ayant une longueur de 15 mm et présentant la même hauteur que la tige du cylindre sur le côté adjacent au cylindre (1) et une hauteur de 8 mm sur le côté éloigné du cylindre (1).

4. Système selon la revendication 3, dans lequel le cylindre (1) présentant un crochet dirigé vers le bas (10) comprend le crochet (10) sous la forme d'une bande large de 5 mm, et ledit alésage fileté (6) pour insérer lesdites vis (7) est situé sur le côté dudit cylindre (1), diamétralement opposé au côté à partir duquel le crochet (10) fait saillie.

5. Système selon la revendication 3, dans lequel le cylindre (1) présentant un crochet dirigé vers le haut (11) comprend le crochet (11) sous la forme d'une bande large de 5 mm, et ledit alésage fileté (6) pour insérer lesdites vis (7) est situé sur le côté dudit cylindre (1), diamétralement opposé au côté à partir duquel le crochet (11) fait saillie.

6. Système selon la revendication 3, dans lequel le cylindre (1) présentant une plaque à vis articulaire (12) présente ladite plaque (12) percée d'un trou (13) présentant un diamètre de 3 mm, ledit trou (13) étant situé à une distance de 2 mm de l'extrémité la plus longue de ladite plaque (13) distante du cylindre (1) et ledit cylindre (1) comprend un alésage fileté (6) supplémentaire pour insérer lesdites vis (7), les deux alésages filetés (6) étant situés à angle droit par rapport à ladite plaque (12).

7. Système selon les revendications 1 à 6, comprenant en outre
- un cylindre de fermeture (15) présentant une hauteur égale à la plus petite desdites hauteurs et comprenant :
- une tige de coupe transversale arrondie, et l'extrémité supérieure dudit cylindre de fermeture (15) étant une hémisphère (2) présentant un diamètre inférieur de 0,5 mm au diamètre de la section transversale dudit cylindre de fermeture (15)
- un alésage circulaire axial (4) présentant un calibre de 2 mm, ledit alésage circulaire axial (4) débouchant au niveau des extrémités inférieure et supérieure dudit cylindre de fermeture (15) pour former un conduit intérieur, l'ouverture supérieure dudit alésage circulaire axial (4) étant entoure de quatre trous (5), lesdits quatre trous (5) étant agencés au niveau des extrémités d'une croix virtuelle, les lignes de raccordement ayant chacune une longueur de 4 mm, et ladite ouverture supérieur se trouvant au niveau de l'intersection desdits lignes de raccordement ;
- une extrémité inférieure plate (16) pour fermer le système ;
- une rainure dièdre (38) le long de la tige, ladite rainure (38) étant dirigée vers l'axe central ; et
- ledit alésage circulaire axial (4) débouchant au niveau de l'extrémité supérieure dudit cylindre de fermeture (15) et devient ladite rainure (38) dans ladite tige du cylindre de fermeture (15).

8. Système selon les revendications 1 à 7, dans lequel
(a) ledit fil tressé (18) est un fil métallique tressé présentant une longueur de 40 cm et une épaisseur de 1,5 mm afin de passer librement à travers l'alésage circulaire axial (4) desdits cylindres (1, 1a) ;
(b) ledit fil tressé (18) est fixé au niveau d'une première extrémité sur une plaque circulaire (9) présentant un diamètre de 4 mm et ayant la forme d'une coquille sphérique, dont la surface est ajustée sur la surface supérieure des cylindres (1, 1a) ;
(c) ladite plaque circulaire (9) comprend quatre pointes (19) chacune faisant saillie, sur une longueur de 1 mm, à partir des extrémités de deux diamètres perpendiculaires de ladite plaque (9) de façon à être logée dans lesdits quatre trous (5) entourant ledit alésage circulaire axial (4) au niveau des extrémités supérieures desdits cylindres (1, 1a) ; et
(d) ledit fil tressé (18) au niveau de sa seconde extrémité se termine en un point (20) permettant audit fil tressé (18) de passer facilement à travers ledit alésage circulaire axial (4) des cylindres (1, 1a)

9. Système selon la revendication 7, dans lequel le moyen de serrage du fil comprend une clé présentant :
(a) une première partie (21) étant un cylindre métallique présentant une longueur de 20 cm et un diamètre de 1 cm, une première extrémité de ladite première partie (21) présentant deux plaquettes incurvées (22) dimensionnées de façon à pouvoir maintenir fermement entre elles le cylindre de fermeture (15) ; la première partie présentant un alésage (23) présentant un calibre de 2mm prévu sur la surface de ladite première partie (21) au milieu desdites deux plaquettes incurvées (22), ledit alésage (23) s'étendant le long de la tige de ladite première partie (21) à nouveau vers la surface (25) à 2 cm de la seconde extrémité de ladite première partie (21) ; une coupelle cylindrique au niveau de la surface de la seconde extrémité de ladite première partie (21), ladite coupelle s'étendant le long de l'axe central, vers le bas, en direction de la première extrémité ; et le périmètre circulaire de la surface au niveau de la seconde extrémité étant une couronne dentée (28) ; et
(b) la clé présentant une seconde partie (27) conformée comme un prisme hexagonal, présentant une tige (26) qui peut être logée dans ladite coupelle de cylindre de la première partie.

10. Système selon la revendication 9, dans lequel ladite seconde partie (27) qui est conformée comme un prisme hexagonal présente un diamètre principal de 6 mm et une longueur de 3 cm, un alésage (29) de 2 mm apparaissant à une distance de 5 mm de sa première extrémité et s'étendant, vers le haut, à l'intérieur dudit prisme vers la surface, à nouveau, sur la même face, à 1 cm en direction de la seconde extrémité.

11. Système selon la revendication 9 ou la revendication 10, dans lequel la tige cylindrique (26), qui fait saillie à partir de la première extrémité dudit prisme (27) et qui s'ajuste dans la coupelle prévue pour ce faire dans la première partie cylindrique, traverse toute la longueur de la première partie, passant à travers la première extrémité de ladite première partie (26a) et présente un léger bossage qui empêche tout déplacement vertical, fixant ainsi, de manière permanente les première et seconde parties, et la seconde extrémité de la partie prismatique étant une couronne dentée agencée pour mettre en opposition les dents sur la première partie.

12. Système selon les revendications 9 ou 10, ledit système comprenant en outre une clé dynamométrique (30) présentant un pas nexagonal (31) adapté au prisme (27) sur la seconde partie et permettant à celle-ci de tourner autour de la première partie, dans le sens des aiguilles d'une montre, le mouvement de retour étant empêché grâce aux couronnes dentées (28) sur les deux parties en about.
